# EUROPEAN PATENT APPLICATION

(11) **EP 3 039 981 A1**
(43) Date of publication of application: **06.07.2016**
(21) Application number: 14841051.7
(22) Date of filing: 01.09.2014
(51) Int. Cl.: A43B 17/00, G08B 21/02

(54) **BLUETOOTH FALL-ALARM INSOLE**

(30) Priority: 30.08.2013 CN 201310390912
(71) Applicant: Guangdong Appscomm Co., Ltd., Guangzhou, Guangdong 510663 (CN)
(72) Inventor: WEI, Qin, Guangzhou Guangdong 510663 (CN); YU, Jianbin, Guangzhou Guangdong 510663 (CN)
(74) Representative: Sebastian, Jens
(86) International application number: PCT/CN2014/085691
(87) International publication number: WO 2015/027955

(57) **Abstract**

The invention discloses a Bluetooth falling-over alarm insole, including an insole body, wherein the insole body is further provided with a Bluetooth falling-over alarm device that judges through the human body's movement posture whether the people falls down and gives an alarm. The invention has the beneficial effects as follows: the volume is small, it is convenient to carry, it is integrated and mounted in the insole, it has no any auxiliary feeling, it conforms to the people's wearing habit, whether the human body is fallen down is judged in combination with the integration of information detected by the acceleration sensor, the pressure sensor and the angle sensor, the accuracy of judgment is high and is more than 95%.

## Description

### TECHNICAL FIELD

The invention relates to a wireless communication alarm device, and to be specific, a Bluetooth falling-over alarm insole.

### BACKGROUND

As the communication technology and sensor technology keep developing and the information society keeps improving, the development of a convenient, reliable falling-over alarm device becomes possible. It is hoped that there is a device capable of automatically giving an alarm when people falls down and takes an accident injury, so as to treat and cure the injured in a timely way.

Nowadays, the aging problem is on the rise, so it is extremely necessary to design a human body falling-over detection and alarm device that is convenient to wear and has no any auxiliary feeling for the elderly population aged more than 65 years or the intellectual disabilities who are inconvenient to walk, and mountaineers, etc.

At present the common technology for falling-over detection is as follows: ①Aiming at the analysis of an audio signal, a falling-over event is judged by analyzing the frequency of vibration caused by impact, it is complicated to mount and is easy to misjudge and the commonality is poor. ② Aiming at the image analysis of video, the motion state of the object is monitored via a camera in a real time, and it is easy to expose out the privacy of a user. ③ based on a wearable detection device, the existing wearable falling-over monitoring is learned by analyzing the acceleration or acquiring the state of some device (such as a rotary apparatus), the requirement for the falling-over condition is high, the identification condition is single, the power consumption is large, and it is easy to misjudge.

### SUMMARY

With regard to the above problems, the invention aims at providing a Bluetooth falling-over alarm insole with rational structure, convenience in use and low misjudgment rate.

In order to achieve the object of the technology, the scheme of the invention is as follows:
A Bluetooth falling-over alarm insole includes an insole body, wherein the insole body is further provided with a Bluetooth falling-over alarm device that judges through the human body's movement posture whether the people falls down and gives an alarm.

Preferably, the Bluetooth falling-over alarm device includes a single chip microcomputer, wherein the single chip microcomputer is connected with a pressure sensor, an angle sensor, an acceleration sensor, a wireless communication module, an automatic alarm module, a wireless communication module, a short message sending module, a battery and a loudspeaker, and receives signals from the pressure sensor, the angle sensor and the acceleration sensor; when a single chip microcomputer receives that the acceleration value measured by the acceleration sensor is more than the acceleration threshold value, and then the angle value data sent from the angle sensor is analyzed; when the current angle value is more than the angle threshold value, and then the pressure value data sent from the pressure sensor is analyzed; and when the pressure value data is zero, the human body falling-over is judged and the alarm is triggered.

Preferably, the loudspeaker is built in the automatic alarm module.

Preferably, the wireless communication module is directly communication with a receiving device with a Bluetooth function and a wireless communication function.

Preferably, the automatic alarm module includes two modes of remote call for help and local call for help, the characteristic of the remote call for help is to be sent to the receiving device through the wireless communication module and transmitted to a service center or a monitor terminal, and the characteristic of the local call for help is to give an voice alarm via the loudspeaker and automatically send the short message to contact rescue if the alarm has no response.

Preferably, the insole body is provided with a gas-tight cabin in the heel, the Bluetooth falling-over alarm device is arranged in the gas-tight cabin, and the opening of the gas-tight cabin is sealed via a sealing bottom cover.

Preferably, the model of the pressure sensor is FSR∼402 and the pressure sensor is used for detecting the data of a pressure applied to the insole due to the change in the human body's posture.

Preferably, the module of the angle sensor is SCA60C, the angle sensor is used for detecting the data of an inclined angle between the human body and the ground according to the change in the angle between the insole and the ground and judging whether the falling-over basis exceeds the scope of the angle threshold value, and the angle threshold value is set as 55°-65°.

Preferably, the model of the acceleration sensor is MMA7260L, and the acceleration sensor is used for detecting the data of the acceleration generated by human body's movement, and the acceleration threshold value is 2g-2.5g.

The invention has the beneficial effects as follows: the volume is small, it is convenient to carry, it is integrated and mounted in the insole, it has no any auxiliary feeling, it conforms to the people's wearing habit, whether the human body is fallen down is judged in combination with the integration of information detected by the acceleration sensor, the pressure sensor and the angle sensor, the accuracy of judgment is high and is more than 95%.

### BRIEF DESCRIPTION OF THE DRAWINGS

Fig. 1 is a schematic structural diagram of a insole of the invention.
Fig. 2 is a schematic diagram of a circuit structure of a Bluetooth falling-over alarm device of the invention.
Fig. 3 is a flow diagram of falling-over judgment of the invention.

### DETAILED DESCRIPTION

The invention will be further described in details hereinafter with reference to the drawings and the specific embodiments.

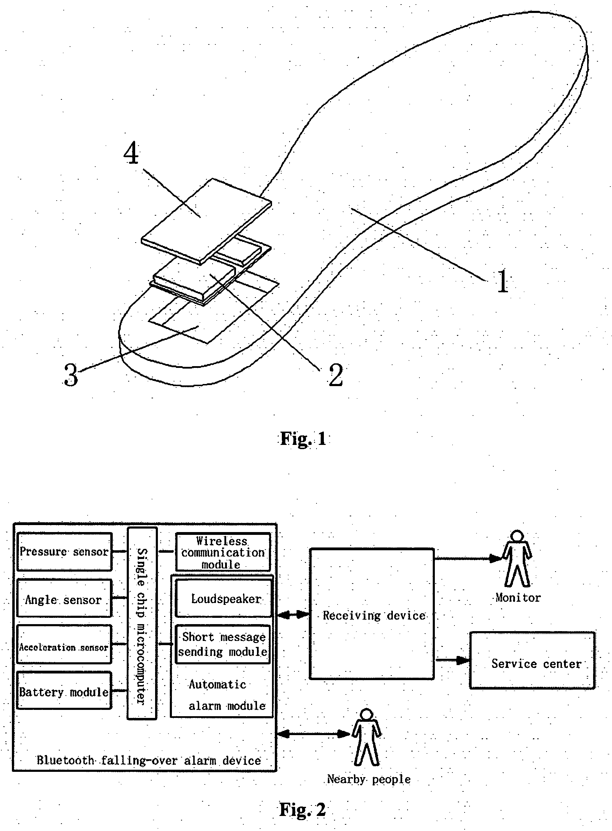

As shown in Fig. 1, the Bluetooth falling-over alarm insole includes an insole body 1 and a Bluetooth falling-over alarm device 2, wherein the insole body 1 is provided with a gas-tight cabin 3 in the heel, the Bluetooth falling-over alarm device 2 is arranged in the gas-tight cabin 3, the opening of the gas-tight cabin is sealed through a sealing bottom cover 4, so as to achieve the waterproof and gas-tight function.

The Bluetooth falling-over alarm device judges whether the people falls down by monitoring the human body's movement posture and gives an alarm. As shown in Fig. 2, it includes a single chip microcomputer, the single chip microcomputer is connected with a pressure sensor, an angle sensor, an acceleration sensor, a wireless communication module, an automatic alarm module, a short message sending module, a loudspeaker and a battery module; the pressure sensor is used for detecting the data of a pressure applied to the insole due to the change in the human body's posture, the angle sensor is used for detecting the data of an inclined angle between the human body and the ground according to the change in the angle between the insole and the ground, the acceleration sensor is used for detecting the data of the acceleration generated by human body's movement and a single chip microcomputer MCU is used for judging whether the human body falls down via an information integration technology of a plurality of sensors. When the human body falls down, the automatic alarm module is automatically triggered, the signal is sent to a receiving device via the wireless communication module and transmitted to a service center or a monitor terminal, at the same time, the loudspeaker is utilized for giving an voice alarm, and a short message is automatically sent for contacting rescue if the alarm has no response. The receiving device is a device with a Bluetooth function and a wireless communication function, such as: mobile phone, Bluetooth bracelet, computer, tablet computer and the like. The battery provides power for the Bluetooth communication device body.

The automatic alarm module is provided with the loudspeaker and a short message sending module. The alarm information can be sent via two modes.
(1) Trigger remote call for help, send the alarm signal to the receiving device through the wireless communication module or the short message sending module, and transmit to the service center or the monitor. The receiving device can be a device with a Bluetooth function and a wireless communication function, such as: mobile phone, Bluetooth bracelet, computer, tablet computer and the like.
(2) Local call for help, broadcast via the loudspeaker to the nearby people for calling for help.

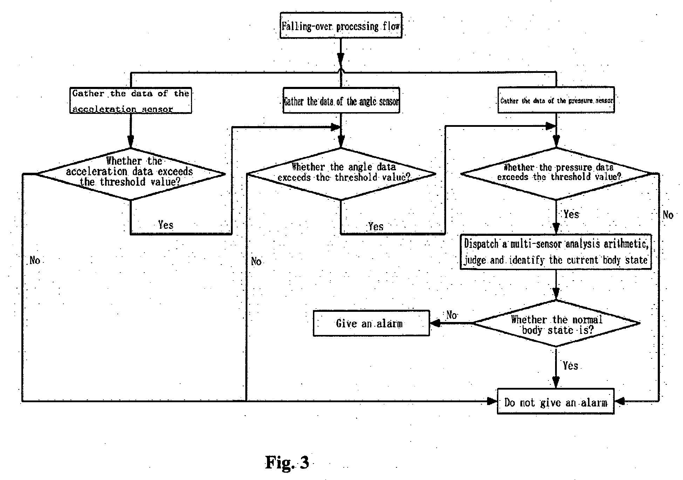

The falling-over alarm flow of the device is as shown in Fig. 3, and the acceleration sensor directly electrified by a power module mainly runs at an energy-saving motion triggering and awakening mode. When the acceleration sensor detects an acceleration threshold value signal that the device wearer exceeds the normal activities, the acceleration sensor outputs an interrupt signal and awakens the single chip microcomputer that is directly electrified by a power supply and is in a low power consumption suspend mode under general conditions; the pressure sensor is in a non-operating state under the normal condition, when the single chip microcomputer is awakened by the acceleration sensor signal, the angle sensor is started by the single chip microcomputer to gather the angle change data, and the pressure sensor is started to gather the pressure change information. As the burst time is less than 2 seconds when the human body falls down instantly, the wearer is judged to fall down when the single chip microcomputer calculates the change in the movement acceleration exceeding the threshold value scope and the change in the posture exceeding the threshold value angle of the device wearer within 2 seconds according to the gathered three-dimensional acceleration change information as well as the pressure data is also changed suddenly.

The multi-sensor integration technology monitors various postures of the human body via the information integration of the acceleration sensor, the tilt sensor and the pressure sensor in a real time, and judges via the gathered acceleration data whether the current acceleration value is more than the acceleration threshold value when falling-over down, the acceleration change Δ threshold value can be set as 2g-2.5g (g is the gravitational acceleration, 1g=9.8N.m/s²), if it is not more than the threshold value, no alarm is given; otherwise, the falling-over posture of the current human body is continuously detected by analyzing the data of the two angle sensors, and whether the current angle is larger than the angle threshold value when falling-over down is judged by analyzing the gather angle data, the angle change Δ threshold value can be set as 55°-65° (about 2/3 of 90°), if it is not more than the threshold value, no alarm is given; otherwise, the gather double-feet pressure data are continuously analyzed, the gravity of the human body is borne by the other parts of the human body after the human body falls down, the soles are left off the ground, the sole pressure value is reduced suddenly or is zero, the human body having been fallen down can be judged at this time and the alarm is triggered. The alarm signal is sent to the emergency processing terminal via the wireless communication module, and a voice alarm is given, and a short message is automatically sent to contact rescue if the alarm has no response.

When the human body takes a rest, such normal activity does not exceed the acceleration threshold value, the reading number of the pressure sensor is zero, the human body activity index as zero can be judged, the system automatically closes the monitoring after 30 minutes, and the single chip microcomputer is in the suspend mode, so as to ensure battery endurance.

The descriptions above are the preferred embodiments of the invention merely, but not intended to limit the invention. Any tiny modification, equivalent replacement and improvement made on the above embodiments according to the technical essence of the invention shall all fall within the protection scope of the technical solutions of the invention.

## Claims

1. A Bluetooth falling-over alarm insole, comprising an insole body, wherein the insole body is further provided with a Bluetooth falling-over alarm device that judges through the human body's movement posture whether the people falls down and gives an alarm.

2. The Bluetooth falling-over alarm insole according to claim 1, wherein the Bluetooth falling-over alarm device comprises a single chip microcomputer, wherein the single chip microcomputer is connected with a pressure sensor, an angle sensor, an acceleration sensor, a wireless communication module, an automatic alarm module, a wireless communication module, a short message sending module, a battery and a loudspeaker, and receives signals from the pressure sensor, the angle sensor and the acceleration sensor; when a single chip microcomputer receives that the acceleration value measured by the acceleration sensor is more than the acceleration threshold value, and then the angle value data sent from the angle sensor is analyzed; when the current angle value is more than the angle threshold value, and then the pressure value data sent from the pressure sensor is analyzed; and when the pressure value data is zero, the human body falling-over is judged and the alarm is triggered.

3. The Bluetooth falling-over alarm insole according to claim 1, wherein the loudspeaker is built in the automatic alarm module.

4. The Bluetooth falling-over alarm insole according to claim 1, wherein the wireless communication module is directly communication with a receiving device with a Bluetooth function and a wireless communication function.

5. The Bluetooth falling-over alarm insole according to claim 1, wherein the insole body is provided with a gas-tight cabin in the heel, the Bluetooth falling-over alarm device is arranged in the gas-tight cabin, and the opening of the gas-tight cabin is sealed via a sealing bottom cover.

6. The Bluetooth falling-over alarm insole according to claim 1, wherein the model of the pressure sensor is FSR-402 and the pressure sensor is used for detecting the data of a pressure applied to the insole due to the change in the human body's posture.

7. The Bluetooth falling-over alarm insole according to claim 1, wherein the module of the angle sensor is SCA60C, the angle sensor is used for detecting the data of an inclined angle between the human body and the ground according to the change in the angle between the insole and the ground and judging whether the falling-over basis exceeds the scope of the angle threshold value, and the angle threshold value is set as 55°-65°.

8. The Bluetooth falling-over alarm insole according to claim 1, wherein the model of the acceleration sensor is MMA7250L, and the acceleration sensor is used for detecting the data of the acceleration generated by human body's movement, and the acceleration threshold value is 2g-2.5g.
